# EUROPEAN PATENT SPECIFICATION

(11) **EP 4 464 284 B1**
(45) Date of publication and mention of the grant of the patent: **18.11.2026**
(21) Application number: 24172552.2
(22) Date of filing: 25.04.2024
(51) Int. Cl.: A61F 2/78, A43C 11/00, A43C 11/16, A61F 5/01, F16G 11/12

(54) **A CABLE TENSIONER FOR A WEARABLE ITEM**
KABELSPANNER FÜR EINEN TRAGBAREN ARTIKEL
TENDEUR DE CÂBLE POUR ARTICLE VESTIMENTAIRE

(30) Priority: 17.05.2023 GB 202307361
(43) Date of publication of application: 20.11.2024
(73) Proprietor: Open Bionics Ltd, Bristol BS1 2NB (GB)
(72) Inventor: WOOD, Steve, Swindon, SN2 1LT (GB)
(74) Representative: Haseltine Lake Kempner LLP

(56) References cited:
- WO-A1-2021/163578
- DE-U1- 202016 104 318
- US-A- 4 633 599
- US-A1- 2008 083 135
- US-A1- 2023 100 239

## Description

### FIELD OF THE INVENTION

The present disclosure relates to a cable tensioner for a wearable item, and particularly, although not exclusively, relates to a cable tensioner for a wearable item with a gear mechanism.

### BACKGROUND OF THE INVENTION

A prosthesis is an artificial body part and EP3545914A1 discloses an example of a prosthetic limb. The prosthetic limb disclosed in EP3545914A1 uses a cable tensioning system to tighten a socket to the end of a user's residual limb and adjust the fit. However, previously-proposed cable tensioners apply tension in a series of gradations and are not capable of fine adjustment. DE202016104318U 1 discloses a cable tensioner for tightening and loosening a cord, comprising: a housing with: a base, a wall extending from the base to form a space with the base, and an annular toothing disposed on the wall and facing the space, a dial covering the housing, a fastening unit disposed inside the space, wherein one end of the fastening unit is connected to the dial, wherein the fastening unit comprises: an arm engaging with the annular toothing to prevent the dial from rotating with respect to the housing in a loosening direction, wherein the arm is radially disengaged from the annular toothing in a tightening direction, which is opposite to the loosening direction, and a spool disposed inside the space, wherein the spool is connected to the fastening unit and is rotated together with the fastening unit, wherein the cord is wound around the spool and connected to the spool, wherein the fastening unit has a first position and a second position with respect to the housing, wherein when the fastening unit is in the first position, the spool is connected to the dial to rotate in the tightening direction to tighten the cord, and wherein when the fastening unit is in the second position, the arm is disengaged from the annular toothing and the cord is pulled out, such that the spool is coupled to the dial to rotate in the loosening direction.

US2023/100239A1, US2008/083135A1, US4633599A and WO2021/163578A1 disclose other previously-proposed tensioning devices.

### SUMMARY OF THE INVENTION

The invention is as defined in claim 1.

The first gear ratio may be greater than the second gear ratio by at least an order of magnitude, e.g. 10 times greater. The first gear ratio may be approximately 10:1 (e.g., 10:1) or higher, for example 12:1. The second gear ratio may be approximately 1:1 (e.g., 1:1).

The gear mechanism may comprise an epicyclic gear arrangement. The epicyclic gear arrangement may comprise at least two gears mounted so that the centre of one gear revolves around the centre of the other.

The fixed portion may comprise a fixed gear. The fixed gear may correspond to a ring gear of the epicyclic gear arrangement. The fixed gear may be an internal gear, e.g., comprising teeth extending radially inward.

The reel may comprise a reel gear. The reel gear may correspond to a sun gear of the epicyclic gear arrangement. The reel gear may be an internal gear, e.g., comprising teeth extending radially inward. The reel gear may be axially spaced apart from the fixed gear (e.g., in the longitudinal direction).

The gear mechanism may comprise a dial gear. The dial gear may rotate with the rotatable dial. The dial gear may be coupled (e.g., rigidly connected) to the rotatable dial. The dial gear may be arranged to selectively engage the reel gear when the rotatable dial is in the second position. The dial gear may be an external gear, e.g., comprising teeth extending radially outward.

The gear mechanism may comprise at least one planet gear. The planet gear may be operatively disposed between the reel gear and the fixed gear. The planet gear may be an external gear, e.g., comprising teeth extending radially outward. The planet gear may be rotatably coupled to the rotatable dial. An axis of rotation of the planet gear relative to the rotatable dial may be eccentric relative to (e.g., spaced apart from) an axis of rotation of the rotatable dial relative to the fixed portion.

The dial gear may be fixedly attached at an end of a shaft. The shaft may be fixedly attached to the rotatable dial. The shaft may be eccentric relative to (e.g., spaced apart from) the axis of rotation of the dial relative to the fixed portion. The planet gear may rotate about the shaft.

The gear mechanism may comprise at least one further planet gear. The further planet gear may be axially spaced apart from the planet gear (e.g., in the longitudinal direction). The further planet gear may be connected to the planet gear such that they rotate together. The further planet gear may rotate about the shaft.

The planet gear may engage (e.g., mesh with) the fixed gear. The further planet gear may engage (e.g. mesh with) the reel gear when the rotatable dial is in the first position. The planet gear may not engage the fixed gear when the when the rotatable dial is in the second position.

The fixed portion may comprise a fixed gear. The reel may comprise a reel gear. The gear mechanism may comprise at least one planet gear and at least one further planet gear. The planet gear and further planet may be axially spaced apart and connected so as to rotate together. The further planet gear may engage the reel gear and the planet gear may selectively engage the fixed gear, e.g., when the rotatable dial is in the first position.

The fixed portion may substantially surround the reel, e.g., such that the reel rotates within the fixed portion. The fixed portion may rotatably support the reel. The fixed portion may comprise at least one opening for the cable to pass through and be wound round the reel.

One of the rotatable dial and the fixed portion may comprise at least one resiliently flexible part. The other of the rotatable dial and the fixed portion may comprise a ridge. The resiliently flexible part and the ridge may be configured to interact together and resiliently resist movement of the rotatable dial between the first and second positions.

The cable tensioner may comprise a base portion to which the fixed portion may be coupled. The base portion or fixed portion may be configured for coupling to the wearable item.

The wearable item may be a prosthesis (e.g., a prosthetic component, such as a limb or part thereof), an orthotic, an item of clothing (such as a shoe, boot or other garment), an item of sports apparel or any other wearable item.

According to another specific aspect, there is provided a wearable item comprising any of the above-mentioned cable tensioners and the cable, the cable tensioner and cable being arranged such that the cable tensioner tightens the cable to tighten the wearable item with respect to a wearer of the wearable item.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Figure 1 is a perspective view of a cable tensioner according to an example of the present disclosure;
Figure 2 is a side view of the cable tensioner according to the example of the present disclosure;
Figures 3a and 3b (collectively Figure 3) are exploded perspective views of the cable tensioner according to the example of the present disclosure with Figure 3a being a top perspective view and Figure 3b being a bottom perspective view;
Figures 4a and 4b (collectively Figure 4) are perspective sectional views of the cable tensioner according to the example of the present disclosure with Figure 4a showing a rotatable dial of the cable tensioner in a first position and Figure 4b showing the rotatable dial in a second position;
Figures 5a and 5b (collectively Figure 5) are top sectional views of the cable tensioner according to the example of the present disclosure in the first position with Figure 5a showing a showing a section through the teeth of a fixed gear and Figure 5b showing a section through the teeth of a reel gear;
Figures 6a and 6b (collectively Figure 6) are top sectional views of the cable tensioner according to the example of the present disclosure in the second position with Figure 6a showing a showing a section through the teeth of the fixed gear and Figure 6b showing a section through the teeth of the reel gear; and
Figure 7 is a perspective of a wearable prosthetic item with the cable tensioner according to the example of the present disclosure.

### DETAILED DESCRIPTION OF EMBODIMENTS

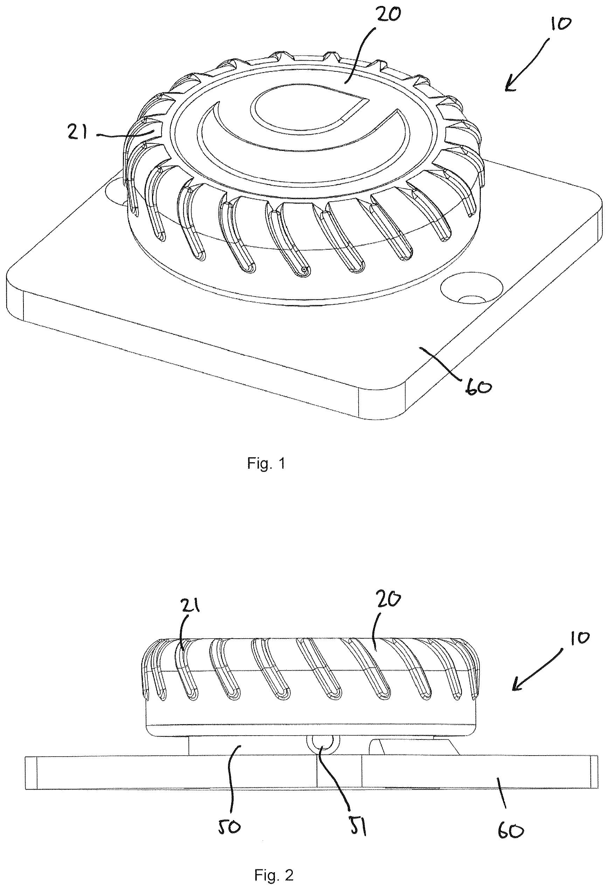

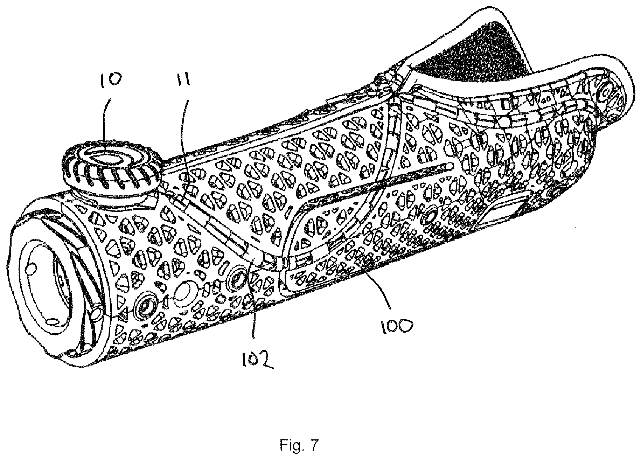

With reference to Figures 1 to 4 and 7, the present disclosure relates to a cable tensioner 10, which may be used for tensioning a cable 11 of a wearable item. In a particular example, as shown in Figure 7, the wearable item may be a prosthesis 100, such as a prosthetic limb. However, in other examples (not shown), the wearable item may be an orthotic, an item of clothing (such as a shoe, boot or other garment), an item of sports apparel or any other wearable item. The cable 11 may be arranged such that a tension in the cable 11 may help secure the wearable item in place, e.g. in a manner similar to that of a lace in a shoe. The cable tensioner 10 may hold the tension in the cable 11.

The cable tensioner 10 comprises a rotatable dial 20, which a user (such as the wearer of the wearable item) may manually adjust, e.g. to adjust the tension in the cable 11. The dial 20 may be substantially tubular with a substantially circular cross-section. An end wall may close one end of the substantially tubular dial. As depicted, the dial 20 may comprise a plurality of grip features 21 (such as indentions or projections) that may improve the user's grip of the dial 20. The grip features 21 may be provided around a circumference of the dial 20.

The cable tensioner 10 further comprises a rotatable reel 30, which is configured to receive the cable and for the cable to be wound around the reel 30. The reel 30 may be substantially tubular with a substantially circular cross-section and may comprise a pair of rims 31a, 31b at opposite ends of the reel. The rims 31a, 31b may help constrain the cable to remain on the reel 30 as it is being wound. The reel 30 may further comprise one or more attachment points 32, to which the cable 11 may be secured.

The cable tensioner 10 further comprises a gear mechanism 40. The gear mechanism 40 rotatably couples the dial 20 and the reel 30 together such that rotation of the dial 20 causes rotation of the reel 30. The gear mechanism 40 may comprise an epicyclic type gear arrangement. The epicyclic gear arrangement may comprise at least two gears mounted so that the centre of one gear revolves around the centre of the other. The gear mechanism 40 will be described in more detail below.

The cable tensioner 10 further comprises a fixed portion 50, which may rotatably support each of the dial 20 and reel 30. The dial 20 and reel 30 may each be rotatable relative to the fixed portion 50. For example, the fixed portion 50 may be substantially tubular with a substantially circular cross-section and may substantially surround the reel 30, e.g., such that the reel 30 rotates within the fixed portion 50. In a similar manner, the dial 20 may substantially surround the fixed portion 50, e.g., such that the dial 20 rotates around the fixed portion 50. The axis of rotation of the dial 20 may coincide with that of the reel 30. The dial 20, reel 30 and fixed portion 50 may be coaxial.

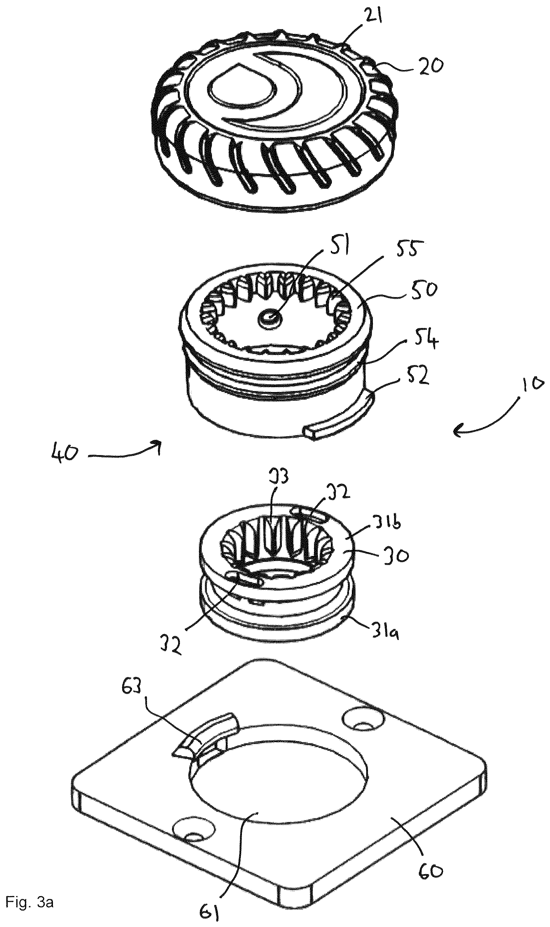

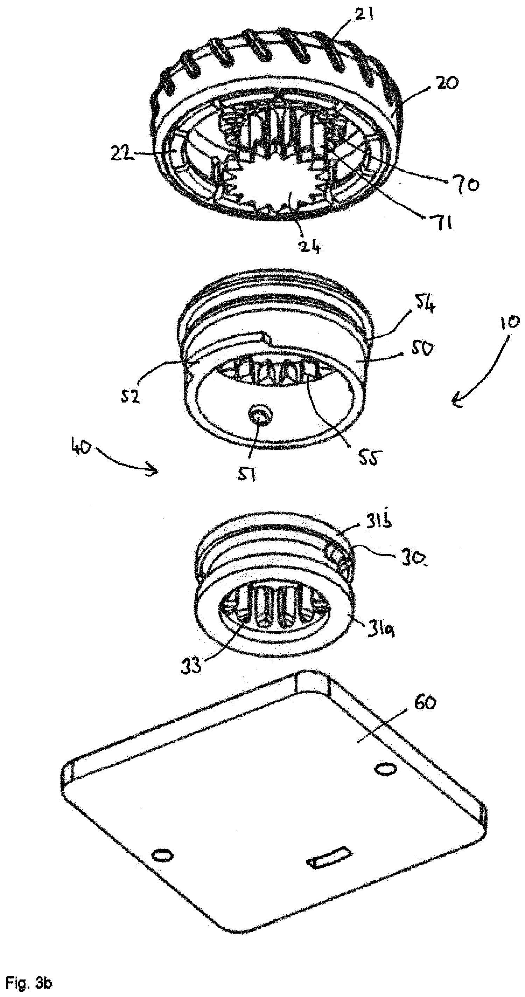

As best depicted in Figures 2 and 3, the fixed portion 50 may comprise at least one opening 51 for the cable to pass through and be wound round the reel 30. The dial 20 may not extend over the opening 51 so that the cable is free to extend beyond the cable tensioner 10.

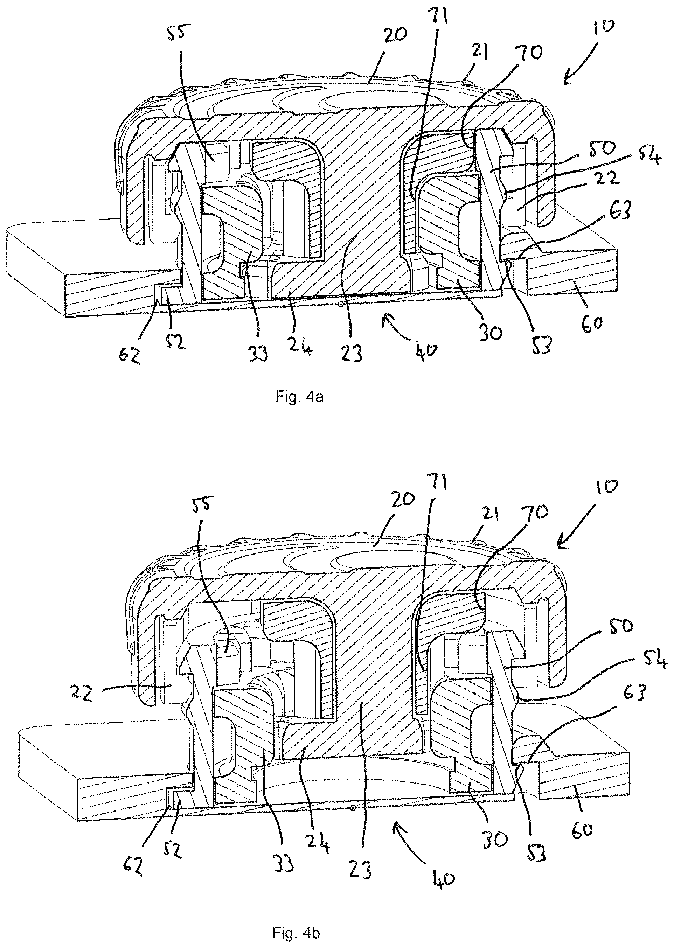

Referring still to Figures 1 to 4, the cable tensioner 10 may further comprise a base portion 60 to which the fixed portion 50 may be coupled. In the example shown, the base portion comprises a depression 61, which may receive the fixed portion 50. The depression 61 maybe substantially circular, e.g. to match an outer profile of the fixed portion 50. As best shown in Figure 4, a tab 52 on a side of the fixed portion 50 may engage a corresponding recess 62, e.g. in a side wall of the depression 61. The fixed portion 50 may further comprise an abutment shoulder 53 that engages a corresponding abutment shoulder 63 on the base portion 60. The tab 52 and recess 62 may be diametrically opposed to the abutment shoulders 53, 63. During assembly the tab 52 may be first engaged in the recess 62 and the abutment shoulders 53, 63 may then ride over one another and provide a snap-fit as the fixed portion 50 is connected with the base portion 60. Once assembled, interactions between the abutment shoulders 53, 63 and between the tab 52 and recess 62 may prevent axial movement of the fixed portion 50 relative to the base portion 60. Further, an interaction between sides of the abutment shoulders 53, 63 and/or between the tab 52 and recess 62 may also prevent relative rotation of the fixed portion 50 and base portion 60. Although separate base portion 60 and fixed portion 50 have been described, it is also envisaged that the base portion 60 and fixed portion 50 may be integral with one another.

The base portion 60 may be configured for coupling to the wearable item. The base portion 60 may be flat or curved, e.g. to suit the shape of the wearable item to which it is to be attached. The cable tensioner 10 may be connected (e.g. via the base portion 60) to the wearable item by virtue of an adhesive, at least one fastener, stitching or any other coupling. For example, the base portion 60 may be sewn to a fabric or other material of the wearable item. The base portion 60 may also be integral with the wearable item. It is also envisaged that the base portion 60 may be omitted and the fixed portion 50 may couple directly to the wearable item, e.g. using similar means.

In addition to being rotatable about the fixed portion 50, the dial 20 may be axially movable relative to the fixed portion 50, i.e. in a longitudinal direction of the cable tensioner 10. Figure 4a shows the dial 20 in a first axial position and Figure 4b shows the dial in a second axial position. The dial 20 may axially slide relative to the fixed portion 50.

As is best shown in Figures 3b and 4, the dial 20 may comprise at least one resiliently flexible part. In the particular example shown, the dial 20 comprises a plurality of fingers 22 circumferentially distributed about the dial 20. Each finger 22 may form a cantilever extending in the axial direction and may resiliently flex. A distal end of each finger 22 may face and contact a surface of the fixed portion 50, thereby facilitating the sliding action in the axial and/or circumferential directions. The fixed portion 50 may comprise a ridge 54 circumferentially disposed about the surface of the fixed portion 50. The resiliently flexible fingers 22 and the ridge 54 may be configured to interact together and resiliently resist movement of the dial 20 between the first and second axial positions. For example, a projection at the distal end of each finger 22 may ride over the ridge 54 and cause the fingers 22 to flex. A force is therefore required by the user to move the dial 20 between the first and second axial positions. It is equally envisaged that the above-described arrangement may be reversed, e.g. with the fixed portion 50 comprising the resiliently flexible part(s) and the dial 20 comprising a corresponding ridge for the flexible part(s) to ride over.

The gear mechanism 40 is configured such that in the first axial position of the dial 20, the gear mechanism has a first gear ratio. The gear mechanism 40 is further configured such that in the second axial position of the dial 20, the gear mechanism has a second gear ratio. (It is also envisaged that the cable tensioner may be reconfigured in other ways to move between the first and second gear ratios, for example by virtue of a manually operated actuator that may act on the gear mechanism to change the gear ratio.) The first and second gear ratios are different, the first gear ratio is greater than the second gear ratio. In particular, more revolutions of the dial 20 may be required for a single turn of the reel 30 when the dial 20 is in the first axial position than when the dial 20 is in the second axial position.

With particular reference to Figures 4 to 6, the gear mechanism 40 will now be described in more detail. The fixed portion 50 may comprise a fixed gear 55. The fixed gear 55 may be an internal gear, e.g., comprising teeth extending radially inward. The fixed gear 55 may correspond to a ring gear of an epicyclic gear arrangement. In a similar manner, the reel 30 may comprise a reel gear 33. The reel gear 33 may be an internal gear, e.g., comprising teeth extending radially inward. The reel gear 33 may correspond to a sun gear of an epicyclic gear arrangement. The reel gear 33 may be axially spaced apart from the fixed gear 55 (e.g., in the longitudinal direction).

In addition to the reel gear 33 and fixed gear 55, the gear mechanism 40 may also comprise at least one planet gear 70. The planet gear 70 is arranged to selectively engage the fixed gear 55 when the dial 20 is in the first axial position. The planet gear 70 may be operatively disposed between the reel gear 33 and the fixed gear 55, e.g. in a gear train between the reel gear 33 and the fixed gear 55. The planet gear 70 may be an external gear, e.g., comprising teeth extending radially outward. The planet gear 70 may be rotatably coupled to the dial 20. An axis of rotation of the planet gear 70 relative to the dial 20 may be eccentric relative to (e.g., spaced apart from) an axis of rotation of the dial 20 relative to the fixed portion 50. In particular, the planet gear 70 may rotate about a shaft 23 and the shaft 23 may be fixedly attached to the dial 20. The shaft 23 may be eccentric relative to (e.g., spaced apart from) the axis of rotation of the dial relative to the fixed portion 50. In other words, the shaft 23 may not be coaxial with the dial 20. Accordingly, a centre of the planet gear 70 revolves around a centre of the dial 20.

The gear mechanism 40 may comprise at least one further planet gear 71. The further planet gear 71 may also be operatively disposed between the reel gear 33 and the fixed gear 55, e.g. in the gear train between the reel gear 33 and the fixed gear 55. The further planet gear 71 may be axially spaced apart from the planet gear 70 (e.g., in the longitudinal direction). The further planet gear 71 may be fixed to the planet gear 70 such that they rotate together, e.g. about the shaft 23. (The further planet gear 71 may simply be an axially spaced apart portion of the planet gear 70.) As such, the further planet gear 71 may also rotate eccentrically to the dial 20. The further planet gear 71 may be an external gear, e.g., comprising teeth extending radially outward. The further planet gear 71 may have fewer teeth that the planet gear 70.

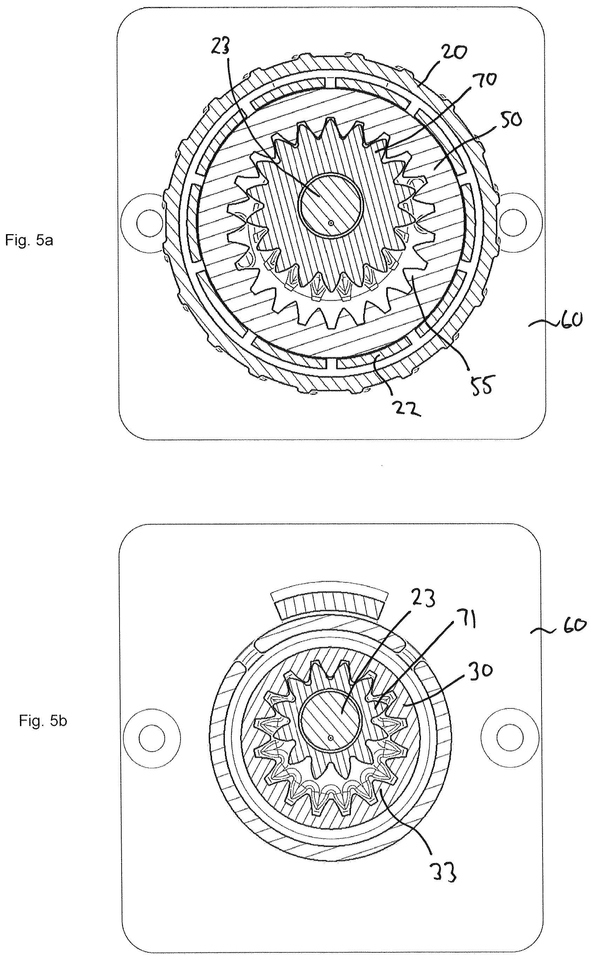

As shown in Figures 4a and 5a, when the dial 20 is in the first axial position, the planet gear 70 may engage (e.g., mesh with) the fixed gear 55. Rotation of the dial 20 causes the axis of rotation of the planet gears 70, 71 to rotate. As the planet gear 70 meshes with the fixed gear 55, this causes rotation of the planet gear 70 about the shaft 23. The further planet gear 71 also then rotates since it is fixed to the planet gear 70. As shown in Figures 4a and 5b, the further planet gear 71 may engage (e.g. mesh with) the reel gear 33 (when the rotatable dial is in the first position). Rotation of the further planet gear 71 (both about shaft 23 and the rotational axis of the dial 20) causes the reel 30 to rotate. This gear train occurs when the dial 20 is in the first axial position and defines the first gear ratio. The axial separation of the reel gear 33 and fixed gear 55 and the arrangement of the planet gears 70, 71 permits a compact gear mechanism 40 with a high gear ratio. The first gear ratio may be approximately 10:1 or higher, for example 12:1. (A gear ratio of 10:1 denotes that the dial 20 turns 10 times for each turn of the reel 30.)

The gear mechanism 40 may further comprise a dial gear 24. The dial gear 24 may be arranged to selectively engage the reel gear 33 when the dial 20 is in the second axial position. The dial gear 24 may be fixed relative to the dial 20, e.g. such that it rotates with the dial 20. The dial gear 24 may be an external gear, e.g., comprising teeth extending radially outward. In contrast to the planet gears 70, 71, a centre of the dial gear 24 may be aligned with a centre of the dial 20. In other words, the dial gear 24 may not be eccentric relative to the dial 20, reel 30 or fixed portion 50. As depicted, the dial gear 24 may be fixedly attached at an end of the shaft 23. The shaft 23 may not be coaxial with the dial gear 24 because the dial gear 24 is coaxial with the dial 20 and the shaft 23 is not coaxial with the dial 20. However, the dial gear 24 may provide a surface that holds the planet gears 70, 71 in place.

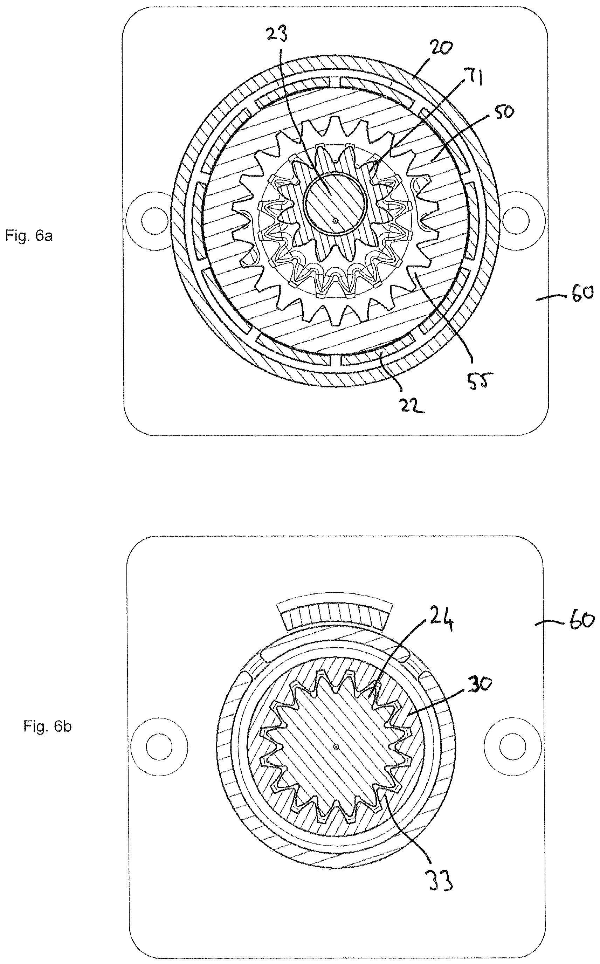

As shown in Figures 4b and 6, when the dial 20 is in the second axial position, the dial gear 24 may engage the reel gear 33. In the first axial position (depicted in Figure 4a), the dial gear 24 is not in mesh with the reel gear 33. However, when the dial 20 is moved into the second position, the dial gear 24 moves into engagement with the reel gear 33 (as depicted in Figure 6b). By contrast, as depicted in Figures 4b and 6a, the planet gear 70 moves out of engagement with the fixed gear 55 when the dial 20 is moved into the second position. The further planet gear 71 may or may not engage the reel gear 33 when the dial 20 is in the second position. The engagement of the further planet gear 71 with the reel gear 33 when the dial 20 is in the second axial position is immaterial because the planet gears 70, 71 are free to rotate due to the planet gear 70 not engaging the fixed gear 55.

As depicted in Figure 6b, the dial gear 24 and reel gear 33 may be coaxial and may have a corresponding number of teeth. As such, the second gear ratio may be 1:1 such that the reel 30 turns in synchrony with the dial 20. In any event, the first gear ratio may be greater than the second gear ratio by at least an order of magnitude.

Although an epicyclic type gear mechanism 40 has been described above, it is also envisaged that other types of gear mechanism could be deployed. For example, the gear mechanism may comprise one or more worm gears or other gear combinations to achieve the desired gear ratios.

Components of the cable tensioner 10 may be 3D printed. In particular, the cable tensioner 10 may be 3D printed in a single printing process. However, the components may alternatively be manufactured separately (whether by 3D printing, moulding or otherwise) and may then be assembled together.

With reference to Figure 7, a wearable item, such as prosthesis 100, may comprise the cable tensioner 10. The cable 11 may be arranged so as to tighten an element of the wearable item. For example, the wearable item may define one or more pathways 102 for the cable 11 to extend through. One or two ends of the cable 11 may be connected to the reel 30 such that rotation of the reel may vary the length of the cable outside the cable tensioner 10. As such, rotation of the cable tensioner 10 tightens the cable 11 to apply a tension to the cable and tighten the wearable item with respect to a wearer of the wearable item.

In the first axial position of the dial 20, the first gear ratio may be such that rotation of the dial 20 permits fine adjustment of the reel 30 and thus the tension in the cable. The first gear ratio is also such that the gear mechanism 40 resistively holds the tension in the cable. In particular, a cable tension force required to turn the gear mechanism in reverse may be greater than a tension force required by the wearable item and that is likely to be encountered during tightening of the cable. The first gear ratio may be set such that the maximum desired cable tension for the wearable item is sufficient to overcome the resistance of the gear mechanism 40 when in the first axial position. This may prevent overtightening of the cable beyond a maximum desired cable tension.

In the second axial position of the dial 20, the second gear ratio may have a value that permits coarse adjustment of the reel and/or that the gear mechanism releases a tension in the cable.

In operation, the user may initially make coarse adjustments to the cable length with the dial 20 in the second position. They may then move the dial 20 into the first position to make fine adjustments to the cable length and tension. The large first gear ratio may then effectively lock the cable in place. When the user wants to release the tension in the cable, the user can move the dial 20 into the second position, at which point the gear ratio changes and the cable tension will be released.

The present invention advantageously provides a compact cable tensioner that allows fine adjustment of the cable length and tension (that is not limited to increase in increments). The present invention also provides an automatic means for locking the cable tension (e.g. without requiring a separate ratchet mechanism). Furthermore, the present invention is intuitive and ergonomic to use and may be readily operated one-handed.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A cable tensioner (10) for a wearable item, wherein rotation of the cable tensioner (10) is configured to tighten a cable to apply a tension to the cable and tighten the wearable item with respect to a wearer of the wearable item, the cable tensioner (10) comprising:
a rotatable dial (20);
a rotatable reel (30) configured to receive the cable (11) and for the cable (11) to be wound around the rotatable reel (30);
a fixed portion (50), the rotatable dial (20) and rotatable reel (30) being rotatable relative to the fixed portion (50); and
a gear mechanism (40) rotatably coupling the rotatable dial (20) and the rotatable reel (30),
wherein the rotatable dial (20) is axially movable relative to the fixed portion (50) between a first position and a second position,
wherein the gear mechanism (40) is configured such that in the first position of the rotatable dial (20) the gear mechanism (40) has a first gear ratio and in the second position of the rotatable dial (20) the gear mechanism (40) has a second gear ratio with the first gear ratio being greater than the second gear ratio, and
wherein the first gear ratio has a value such that rotation of the rotatable dial (20) permits fine adjustment of the rotatable reel (30) and that the gear mechanism (40) resistively holds the tension in the cable (11) and the second gear ratio has a value that permits coarse adjustment of the reel (30) and that the gear mechanism (40) releases a tension in the cable (11).

2. The cable tensioner (10) of claim 1, wherein the first gear ratio is greater than the second gear ratio by at least an order of magnitude.

3. The cable tensioner (10) of claim 1 or 2, wherein the first gear ratio is approximately 12:1 and/or the second gear ratio is approximately 1:1.

4. The cable tensioner (10) of any of the preceding claims, wherein the gear mechanism (40) comprises an epicyclic gear arrangement.

5. The cable tensioner (10) of any of the preceding claims, wherein the fixed portion (50) comprises a fixed gear (55).

6. The cable tensioner (10) of any of the preceding claims, wherein the reel (30) comprises a reel gear (33).

7. The cable tensioner (10) of claim 6, wherein the gear mechanism (40) comprises a dial gear (24), the dial gear (24) being arranged to rotate with the rotatable dial (20) and selectively engage the reel gear (33) when the rotatable dial (20) is in the second position.

8. The cable tensioner (10) of any of the preceding claims, wherein the gear mechanism (40) comprises at least one planet gear (70).

9. The cable tensioner (10) of claim 8, wherein the planet gear (70) is rotatably coupled to the rotatable dial (20) and an axis of rotation of the planet gear (70) relative to the rotatable dial (20) is eccentric relative to an axis of rotation of the rotatable dial (20) relative to the fixed portion (50).

10. The cable tensioner (10) of claim 8 or 9, wherein the gear mechanism (40) comprises at least one further planet gear (71), the further planet gear (71) being axially spaced apart from the planet gear (70) and rotating together with the planet gear (70).

11. The cable tensioner (10) of any of the preceding claims, wherein the fixed portion (50) comprises a fixed gear (55), the reel (30) comprises a reel gear (33), and the gear mechanism (40) comprises at least one planet gear (70) and at least one further planet gear (71), wherein the planet gear (70) and further planet are axially spaced apart and connected so as to rotate together, wherein the further planet gear (71) engages the reel gear (33) and the planet gear (70) selectively engages the fixed gear (55) when the rotatable dial (20) is in the first position.

12. The cable tensioner (10) of any of the preceding claims, wherein the fixed portion (50) substantially surrounds the reel (30) such that the reel (30) rotates within the fixed portion (50).

13. The cable tensioner (10) of any of the preceding claims, wherein one of the rotatable dial (20) and the fixed portion (50) comprise at least one resiliently flexible part and the other of the rotatable dial (20) and the fixed portion (50) comprise a ridge (54), the resiliently flexible part and ridge (54) being configured to interact together and resiliently resist movement of the rotatable dial (20) between the first and second positions.

14. The cable tensioner (10) of any of the preceding claims, wherein the cable tensioner (10) comprises a base portion (60) to which the fixed portion (50) is coupled, the base portion (60) being configured for coupling to the wearable item.

15. A wearable item comprising the cable tensioner (10) of any of the preceding claims and the cable (11), the cable tensioner (10) and cable (11) being arranged such that the cable tensioner (10) tightens the cable (11) to tighten the wearable item with respect to a wearer of the wearable item.

## Patentansprüche

1. Kabelspanner (10) für einen tragbaren Artikel, wobei eine Drehung des Kabelspanners (10) konfiguriert ist, um ein Kabel zu spannen, um eine Spannung an das Kabel anzulegen und den tragbaren Artikel in Bezug auf einen Träger des tragbaren Artikels zu spannen, wobei der Kabelspanner (10) Folgendes umfasst:
ein drehbares Einstellrad (20);
eine drehbare Spule (30), die konfiguriert ist, um das Kabel (11) aufzunehmen, und damit das Kabel (11) um die drehbare Spule (30) gewickelt wird;
einen feststehenden Abschnitt (50), wobei das drehbare Einstellrad (20) und die drehbare Spule (30) relativ zu dem feststehenden Abschnitt (50) drehbar sind; und
einen Getriebemechanismus (40), der das drehbare Einstellrad (20) und die drehbare Spule (30) drehbar koppelt,
wobei das drehbare Einstellrad (20) relativ zu dem feststehenden Abschnitt (50) zwischen einer ersten Position und einer zweiten Position axial beweglich ist,
wobei der Getriebemechanismus (40) so konfiguriert ist, dass in der ersten Position des drehbaren Einstellrads (20) der Getriebemechanismus (40) ein erstes Übersetzungsverhältnis aufweist und in der zweiten Position des drehbaren Einstellrads (20) der Getriebemechanismus (40) ein zweites Übersetzungsverhältnis aufweist, wobei das erste Übersetzungsverhältnis größer als das zweite Übersetzungsverhältnis ist, und
wobei das erste Übersetzungsverhältnis einen solchen Wert aufweist, dass eine Drehung des drehbaren Einstellrads (20) eine Feineinstellung der drehbaren Spule (30) zulässt und dass der Getriebemechanismus (40) die Spannung in dem Kabel (11) widerstehend hält, und das zweite Übersetzungsverhältnis einen Wert aufweist, der eine Grobeinstellung der Spule (30) zulässt und dass der Getriebemechanismus (40) eine Spannung in dem Kabel (11) löst.

2. Kabelspanner (10) nach Anspruch 1, wobei das erste Übersetzungsverhältnis um mindestens eine Größenordnung größer als das zweite Übersetzungsverhältnis ist.

3. Kabelspanner (10) nach Anspruch 1 oder 2, wobei das erste Übersetzungsverhältnis ungefähr 12:1 ist und/oder das zweite Übersetzungsverhältnis ungefähr 1:1 ist.

4. Kabelspanner (10) nach einem der vorhergehenden Ansprüche, wobei der Getriebemechanismus (40) eine Planetengetriebeanordnung umfasst.

5. Kabelspanner (10) nach einem der vorhergehenden Ansprüche, wobei der feststehende Abschnitt (50) ein feststehendes Zahnrad (55) umfasst.

6. Kabelspanner (10) nach einem der vorhergehenden Ansprüche, wobei die Spule (30) ein Spulenzahnrad (33) umfasst.

7. Kabelspanner (10) nach Anspruch 6, wobei der Getriebemechanismus (40) ein Einstellrad-Zahnrad (24) umfasst, wobei das Einstellrad-Zahnrad (24) angeordnet ist, um sich mit dem drehbaren Einstellrad (20) zu drehen und selektiv in das Spulenzahnrad (33) einzugreifen, wenn das drehbare Einstellrad (20) in der zweiten Position ist.

8. Kabelspanner (10) nach einem der vorhergehenden Ansprüche, wobei der Getriebemechanismus (40) mindestens ein Planetenzahnrad (70) umfasst.

9. Kabelspanner (10) nach Anspruch 8, wobei das Planetenzahnrad (70) drehbar mit dem drehbaren Einstellrad (20) gekoppelt ist und eine Drehachse des Planetenzahnrads (70) relativ zu dem drehbaren Einstellrad (20) exzentrisch relativ zu einer Drehachse des drehbaren Einstellrads (20) relativ zu dem feststehenden Abschnitt (50) ist.

10. Kabelspanner (10) nach Anspruch 8 oder 9, wobei der Getriebemechanismus (40) mindestens ein weiteres Planetenzahnrad (71) umfasst, wobei das weitere Planetenzahnrad (71) axial von dem Planetenzahnrad (70) beabstandet ist und sich zusammen mit dem Planetenzahnrad (70) dreht.

11. Kabelspanner (10) nach einem der vorhergehenden Ansprüche, wobei der feststehende Abschnitt (50) ein feststehendes Zahnrad (55) umfasst, die Spule (30) ein Spulenzahnrad (33) umfasst und der Getriebemechanismus (40) mindestens ein Planetenzahnrad (70) und mindestens ein weiteres Planetenzahnrad (71) umfasst, wobei das Planetenzahnrad (70) und der weitere Planet axial beabstandet sind und so verbunden sind, dass sie sich zusammen drehen, wobei das weitere Planetenzahnrad (71) in das Spulenzahnrad (33) eingreift und das Planetenzahnrad (70) selektiv in das feststehende Zahnrad (55) eingreift, wenn das drehbare Einstellrad (20) in der ersten Position ist.

12. Kabelspanner (10) nach einem der vorhergehenden Ansprüche, wobei der feststehende Abschnitt (50) die Spule (30) im Wesentlichen umgibt, so dass sich die Spule (30) innerhalb des feststehenden Abschnitts (50) dreht.

13. Kabelspanner (10) nach einem der vorhergehenden Ansprüche, wobei eines von dem drehbaren Einstellrad (20) und dem feststehenden Abschnitt (50) mindestens ein elastisch flexibles Teil umfasst und das andere von dem drehbaren Einstellrad (20) und dem feststehenden Abschnitt (50) eine Erhebung (54) umfasst, wobei das elastisch flexible Teil und die Erhebung (54) konfiguriert sind, um zusammenzuwirken und einer Bewegung des drehbaren Einstellrads (20) zwischen der ersten und der zweiten Position elastisch zu widerstehen.

14. Kabelspanner (10) nach einem der vorhergehenden Ansprüche, wobei der Kabelspanner (10) einen Basisabschnitt (60) umfasst, mit dem der feststehende Abschnitt (50) gekoppelt ist, wobei der Basisabschnitt (60) zum Koppeln mit dem tragbaren Artikel konfiguriert ist.

15. Tragbarer Artikel, umfassend den Kabelspanner (10) nach einem der vorhergehenden Ansprüche und das Kabel (11), wobei der Kabelspanner (10) und das Kabel (11) so angeordnet sind, dass der Kabelspanner (10) das Kabel (11) spannt, um den tragbaren Artikel in Bezug auf einen Träger des tragbaren Artikels zu spannen.

## Revendications

1. Tendeur de câble (10) pour article vestimentaire, dans lequel la rotation du tendeur de câble (10) est configurée pour tendre un câble afin d'appliquer une tension au câble et serrer l'article vestimentaire par rapport à un porteur de l'article vestimentaire, le tendeur de câble (10) comprenant :
un cadran pouvant tourner (20) ;
une bobine pouvant tourner (30) configurée pour recevoir le câble (11) et afin que le câble (11) soit enroulé autour de la bobine pouvant tourner (30) ;
une portion fixe (50), le cadran pouvant tourner (20) et la bobine pouvant tourner (30) étant rotatifs par rapport à la portion fixe (50) ; et
un mécanisme d'engrenage (40) couplant de manière rotative le cadran pouvant tourner (20) et la bobine pouvant tourner (30),
dans lequel le cadran pouvant tourner (20) est mobile axialement par rapport à la portion fixe (50) entre une première position et une seconde position,
dans lequel le mécanisme d'engrenage (40) est configuré de sorte que dans la première position du cadran pouvant tourner (20), le mécanisme d'engrenage (40) présente un premier rapport d'engrenage et dans la seconde position du cadran pouvant tourner (20), le mécanisme d'engrenage (40) présente un second rapport d'engrenage, le premier rapport d'engrenage étant supérieur au second rapport d'engrenage, et
dans lequel le premier rapport d'engrenage présente une valeur telle que la rotation du cadran pouvant tourner (20) permet un réglage fin de la bobine pouvant tourner (30) et que le mécanisme d'engrenage (40) maintient de manière résistive la tension dans le câble (11) et que le second rapport d'engrenage présente une valeur qui permet un réglage grossier de la bobine (30) et que le mécanisme d'engrenage (40) libère une tension dans le câble (11).

2. Tendeur de câble (10) selon la revendication 1, dans lequel le premier rapport d'engrenage est supérieur au second rapport d'engrenage d'au moins un ordre de grandeur.

3. Tendeur de câble (10) selon la revendication 1 ou la revendication 2, dans lequel le premier rapport d'engrenage est d'environ 12:1 et/ou le second rapport d'engrenage est d'environ 1:1.

4. Tendeur de câble (10) selon l'une quelconque des revendications précédentes, dans lequel le mécanisme d'engrenage (40) comprend un agencement d'engrenage épicycloïdal.

5. Tendeur de câble (10) selon l'une quelconque des revendications précédentes, dans lequel la portion fixe (50) comprend un engrenage fixe (55).

6. Tendeur de câble (10) selon l'une quelconque des revendications précédentes, dans lequel la bobine (30) comprend un engrenage de bobine (33).

7. Tendeur de câble (10) selon la revendication 6, dans lequel le mécanisme d'engrenage (40) comprend un engrenage de cadran (24), l'engrenage de cadran (24) étant agencé pour tourner avec le cadran pouvant tourner (20) et entrer en contact sélectivement avec l'engrenage de bobine (33) lorsque le cadran pouvant tourner (20) est dans la seconde position.

8. Tendeur de câble (10) selon l'une quelconque des revendications précédentes, dans lequel le mécanisme d'engrenage (40) comprend au moins un engrenage planétaire (70).

9. Tendeur de câble (10) selon la revendication 8, dans lequel l'engrenage planétaire (70) est couplé de manière rotative au cadran pouvant tourner (20) et un axe de rotation de l'engrenage planétaire (70) par rapport au cadran pouvant tourner (20) est excentré par rapport à un axe de rotation du cadran pouvant tourner (20) par rapport à la portion fixe (50).

10. Tendeur de câble (10) selon la revendication 8 ou la revendication 9, dans lequel le mécanisme d'engrenage (40) comprend au moins un autre engrenage planétaire (71), l'autre engrenage planétaire (71) étant espacé axialement de l'engrenage planétaire (70) et tournant avec l'engrenage planétaire (70).

11. Tendeur de câble (10) selon l'une quelconque des revendications précédentes, dans lequel la portion fixe (50) comprend un engrenage fixe (55), la bobine (30) comprend un engrenage de bobine (33), et le mécanisme d'engrenage (40) comprend au moins un engrenage planétaire (70) et au moins un autre engrenage planétaire (71), dans lequel l'engrenage planétaire (70) et l'autre engrenage planétaire sont espacés axialement et connectés de manière à tourner ensemble, dans lequel l'autre engrenage planétaire (71) entre en contact avec l'engrenage de bobine (33) et l'engrenage planétaire (70) entre en contact sélectivement avec l'engrenage fixe (55) lorsque le cadran pouvant tourner (20) est dans la première position.

12. Tendeur de câble (10) selon l'une quelconque des revendications précédentes, dans lequel la portion fixe (50) entoure sensiblement la bobine (30) de sorte que la bobine (30) tourne à l'intérieur de la portion fixe (50).

13. Tendeur de câble (10) selon l'une quelconque des revendications précédentes, dans lequel l'un ou l'une du cadran pouvant tourner (20) et de la portion fixe (50) comprend au moins une partie élastiquement flexible, et l'autre cadran pouvant tourner (20) et la portion fixe (50) comprennent une arête (54), la partie élastiquement flexible et l'arête (54) étant configurées pour interagir et résister élastiquement au mouvement du cadran pouvant tourner (20) entre les première et seconde positions.

14. Tendeur de câble (10) selon l'une quelconque des revendications précédentes, dans lequel le tendeur de câble (10) comprend une portion de base (60) à laquelle la portion fixe (50) est couplée, la portion de base (60) étant configurée pour se coupler à l'article vestimentaire.

15. Article vestimentaire comprenant le tendeur de câble (10) selon l'une quelconque des revendications précédentes et le câble (11), le tendeur de câble (10) et le câble (11) étant agencés de sorte que le tendeur de câble (10) serre le câble (11) pour serrer l'article vestimentaire par rapport à un porteur de l'article vestimentaire.
